# DEMANDE DE BREVET EUROPEEN

(11) **EP 4 295 803 A1**
(43) Date de publication de la demande: **27.12.2023**
(21) Numéro de dépôt: 23181115.9
(22) Date de dépôt: 23.06.2023
(51) Int. Cl.: A61B 90/70, A61C 19/00, A61B 90/00

(54) **DISPOSITIF DE NETTOYAGE POUR INSTRUMENTS DENTAIRES, MEDICAUX OU CHIRURGICAUX**

(30) Priorité: 23.06.2022 WO PCT/IB2022/055829
(71) Demandeur: Produits Dentaires S.A., 1800 Vevey (CH)
(72) Inventeur: PATEL, Shanon, 1800 VEVEY (CH); OLLIERIC, Mel, 1800 Vevey (CH); GEHRIG, Nicolas, 1800 Vevey (CH)
(74) Mandataire: Micheli & Cie SA

(57) **Abrégé**

La présente invention a pour objet dispositif (1) de nettoyage pour instruments dentaires, médicaux ou chirurgicaux comprenant un support (3), un réceptacle (4) monté sur le support (3) et destiné à recevoir au moins la partie active d'un instrument dentaire, médical ou chirurgical et un couvercle (7) agencé pour être monté de manière amovible sur une extrémité (41) du réceptacle (4). Le couvercle (7) comprend au moins une fente (9), ladite fente (9) étant agencée de sorte qu'au moins la partie active de l'instrument peut être insérée dans le réceptacle (4) et retirée du réceptacle (4) à travers la fente (9), le frottement entre la partie active de l'instrument et au moins une paroi de la fente (9) lorsque ledit instrument traverse ladite fente (9) permettant de nettoyer mécaniquement ladite partie active en retenant débris et saleté liquides ou solides.

## Description

La présente invention a pour objet un dispositif de nettoyage pour instruments dentaires, médicaux ou chirurgicaux.

Durant un traitement dentaire, endodontique ou parodontique, des débris ou des liquides plus ou moins visqueux s'amassent le long des instruments utilisés (fraise, lime, curette ou détartreur). Une possibilité pour le nettoyage des instruments est de prévoir un chiffon disposé sur un plateau sur lequel le praticien vient essuyer la partie active de son instrument pour la nettoyer. Dans le but d'augmenter le confort pour le praticien, des dispositifs ont été imaginés pour se placer sur un doigt ou la main du praticien opposée à la main qui tient l'instrument. Le dispositif peut notamment prendre la forme d'une bague comportant une zone de nettoyage sur laquelle il est possible de venir frotter la partie active de l'instrument. Un tel dispositif est par exemple décrit dans US 2008/0311543. Cette méthode de nettoyage qui consiste à frotter la partie active de l'instrument sur une surface nettoyante est particulièrement adaptée aux instruments du type curette ou détartreur.

Il existe également des dispositifs en forme de bague que le praticien peut enfiler à un de ses doigts et qui comprennent un support destiné à recevoir une mousse dans laquelle peuvent être piqués les instruments prévus pour le traitement dentaire. Ainsi, les instruments sont immédiatement accessibles pour le praticien et, en retirant un instrument de la mousse, les débris qui s'étaient attachés audit instrument restent prisonniers de la mousse, ce qui permet de nettoyer l'instrument. Pour parfaire le nettoyage, il est également possible d'imbiber la mousse d'un produit désinfectant. Le document US2011229843 décrit par exemple un tel dispositif.

Ces dispositifs offrent donc une solution pour le nettoyage des instruments, notamment les instruments endodontiques, mais également une solution pour l'accès et le stockage des instruments durant un traitement et en particulier un traitement dentaire. En effet, durant un traitement dentaire et endodontique notamment, le praticien a recours à plusieurs instruments pour l'accès, la mise en forme, le nettoyage et l'élargissement du canal radiculaire dentaire. Ces instruments sont utilisés séquentiellement selon leur type et leur taille. La séquence d'instruments est déterminée selon le cas à traiter et peut être préparée à l'avance par le praticien lui-même ou une autre personne. Un accès facile à ces instruments est important, pour permettre notamment au praticien de changer d'instrument rapidement et confortablement durant le traitement. Les dispositifs décrits plus haut sous forme d'une bague avec un coussinet en mousse dans lequel piquer les instruments servent donc de stockage des instruments et facilitent la préparation des instruments et leur accès durant un traitement. Cette solution est notamment plus confortable et ergonomique que la simple disposition des instruments sur un plateau.

Cependant, l'utilisation d'un coussinet en mousse dans lequel piquer les instruments n'est pas sans problème : d'une part, il n'est pas facile dans un tel coussinet de distinguer des emplacements précis pour les instruments, ce qui fait courir le risque de perdre l'ordre des instruments pour le traitement ou de mélanger instruments propres et sales durant le traitement. En outre, les instruments endodontiques sont en général effilés et très fins sur leur partie active et il y a un risque de casser ou de plier ladite partie active de l'instrument en le piquant dans la mousse. Or, cette mousse doit être suffisamment rigide pour retenir l'instrument et participer à son nettoyage mécanique lorsque l'instrument est retiré du coussinet. De plus, en enfonçant la partie active d'un instrument sale dans le coussinet en mousse, les débris sont susceptibles de remonter le long de l'instrument ce qui n'est pas souhaitable.

La problématique du nettoyage des instruments et/ou de leur mise à disposition se pose également durant les traitements parodontiques (détartrage inclus) ou d'autres traitements dentaires, médicaux ou chirurgicaux durant lesquels des instruments doivent être nettoyés régulièrement et/ou stockés à portée de main de l'utilisateur.

Le but de la présente invention est de fournir un dispositif de nettoyage pour instruments dentaires, médicaux ou chirurgicaux qui soit efficace, ergonomique, facile d'utilisation et facilement usinable. Un autre but de la présente invention est de fournir un dispositif qui permette en outre la mise à disposition et le stockage aisés, efficients et ergonomiques des instruments, le dispositif permettant de servir de support pour au moins un mais de préférence une séquence d'instruments nécessaires durant un traitement dentaire, médical ou chirurgical.

La présente invention a pour objet un dispositif de nettoyage pour instruments dentaires, médicaux ou chirurgicaux selon la revendication 1.

Les dessins annexés illustrent schématiquement et à titre d'exemple plusieurs formes d'exécution de l'invention.
Les figures 1 à 3 illustre un dispositif de nettoyage pour instruments dentaires, médicaux ou chirurgicaux selon une première forme d'exécution de l'invention vu de côté, de dessus et de dessous respectivement.
La figure 4 illustre une partie du dispositif de la figure 1 vu de côté.
Les figures 5, 6 et 7a et 7b illustrent le couvercle du dispositif selon les figures précédentes vu du dessus, de dessous, de côté et en coupe respectivement.
La figure 8 illustre une variante du dispositif de la figure 3 vue de dessous.
La figure 9a est similaire à la figure 1 tandis que la figure 9b illustre le dispositif de la figure 9a dans une position intermédiaire dans laquelle ledit dispositif peut être facilement positionné sur un doigt par un utilisateur.
Les figures 10 à 12 illustrent une seconde forme d'exécution du dispositif de nettoyage pour instruments dentaires, médicaux ou chirurgicaux selon l'invention vu de côté, de dessus et en coupe respectivement.
Les figures 13 à 17 illustrent une troisième forme d'exécution du dispositif de nettoyage pour instruments dentaires médicaux ou chirurgicaux selon l'invention.

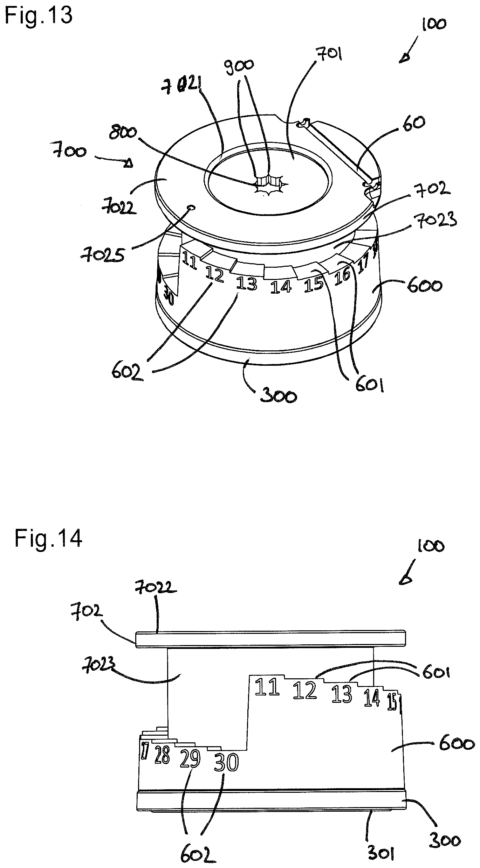

Les figures 13 et 14 illustrent le dispositif vu en perspective et de côté.

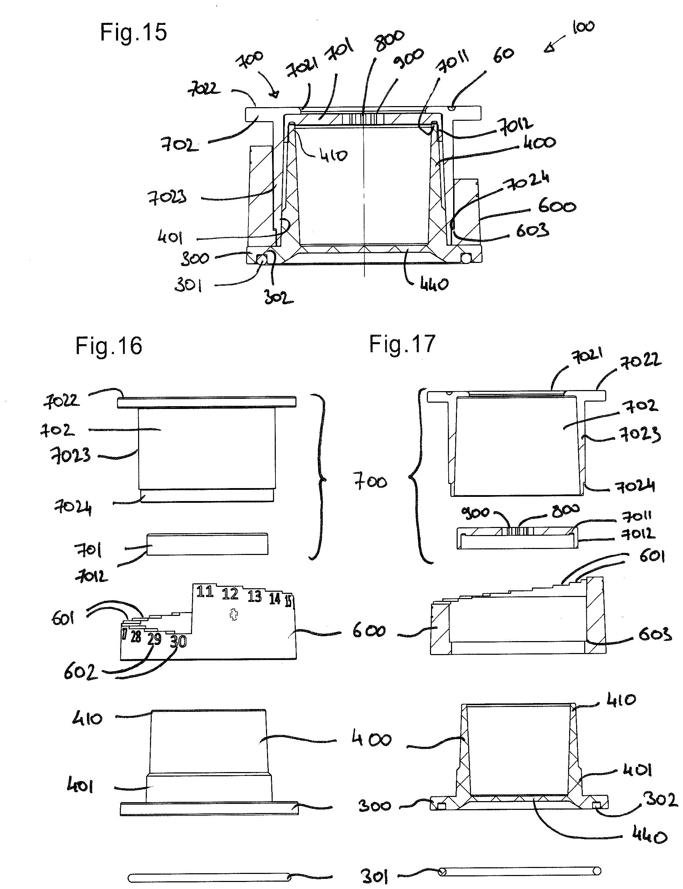

La figure 15 est une vue en coupe de la figure 14.
Les figures 16 et 17 sont des vues éclatées, la figure 17 étant une vue en coupe.

Le dispositif selon l'invention est notamment destiné au nettoyage d'instruments dentaires, médicaux ou chirurgicaux destinés notamment à gratter, fraiser, couper, débrider, exciser, nettoyer... Un tel instrument comprend en général une première portion destinée à être prise en main par un utilisateur ou à être insérée dans un autre outil (manche, contre-angle, moteur...). Cette première portion n'est en générale pas active, c'est-à-dire qu'elle ne participe pas au travail pour lequel l'instrument est conçu. L'instrument comprend en outre une partie active s'étendant à la suite de la première portion et présentant des moyens permettant d'effectuer le travail ou traitement pour lequel l'instrument est conçu. Par exemple, cette partie active peut présenter des arêtes de coupe, un crochet, une gouge, une petite boule de fraisage... Dans le cas d'un instrument endodontique, cette partie active, qui peut être effilée ou non, présente un diamètre relativement fin.

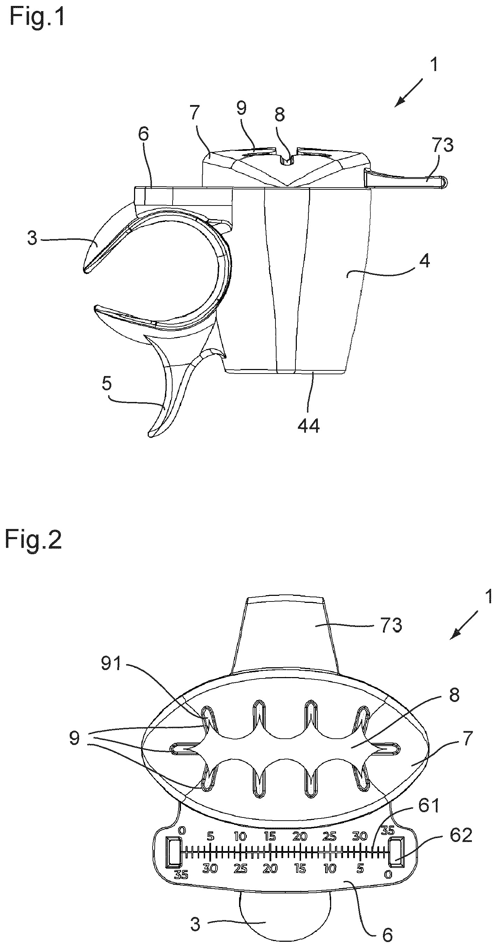

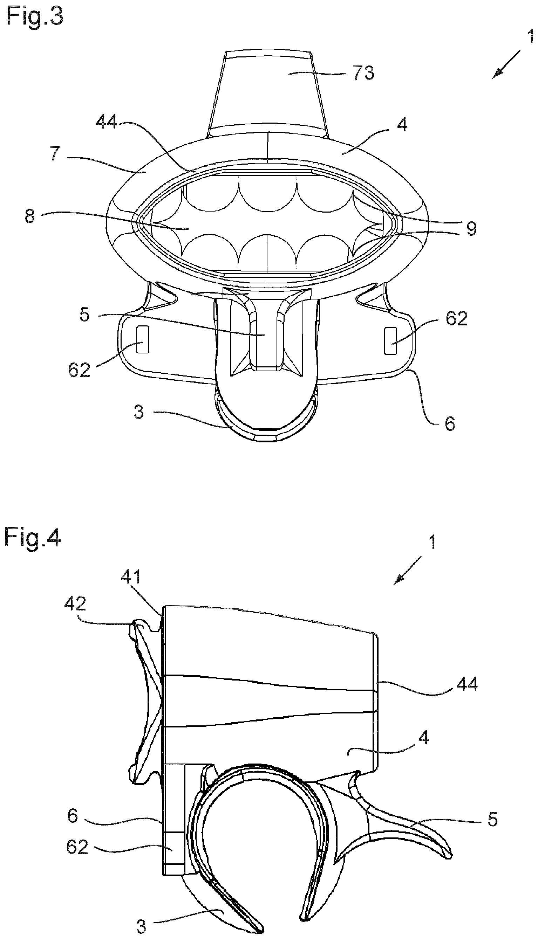

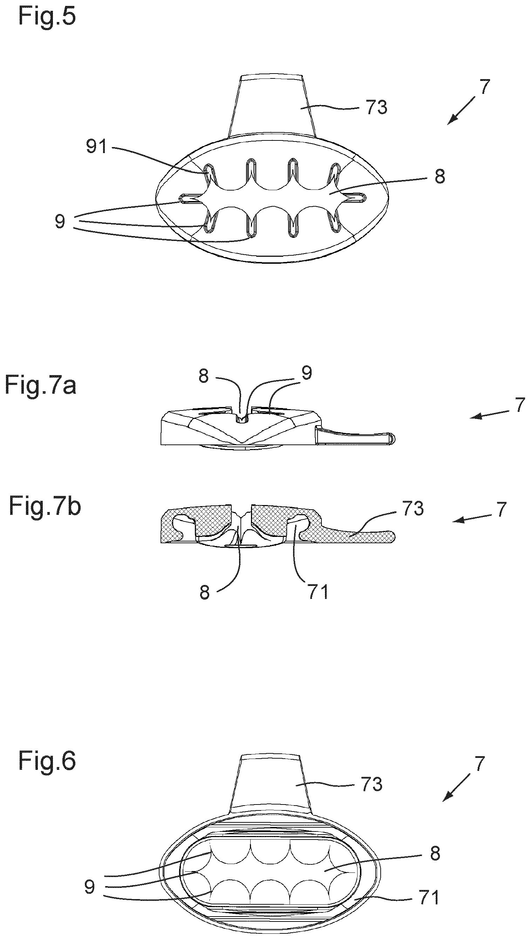

Dans la première forme d'exécution de l'invention telle qu'illustrée aux figures 1à 9b, le dispositif de nettoyage 1 selon l'invention est destiné à un usage dans le domaine dentaire pour des instruments endodontiques ou parodontiques.

Le dispositif 1 est destiné à être positionné sur un doigt d'une main d'un utilisateur durant un traitement dentaire et en particulier un traitement endodontique. Le dispositif 1 est destiné à recevoir un ou plusieurs instruments dentaires pour leur mise à disposition et pour leur nettoyage durant le traitement.

De manière générale, le dispositif de nettoyage selon l'invention comprend un support sur lequel est monté un réceptacle. Dans la première forme d'exécution, le dispositif 1 est destiné à être enfilé sur le doigt d'un utilisateur et le support a donc la forme d'un anneau 3 dans lequel passer un doigt. Tel qu'illustré, l'anneau 3 est ouvert mais en variante cet anneau pourrait être fermé. De préférence, l'anneau 3 comprend en outre une extension 5 agencée pour éviter que le dispositif 1 ne pivote une fois que ledit dispositif 1 est installé sur le doigt d'un utilisateur.

Dans la première forme d'exécution illustrée aux figures 1 à 7b, le réceptacle 4 est ouvert à ses deux extrémités. Sa forme, et en particulier sa longueur, sont telles qu'il est apte à recevoir l'instrument dentaire, et notamment sa partie active en entier et de préférence, une partie au moins de la première portion de l'instrument.

Selon l'invention, le dispositif 1 comprend en outre un couvercle 7 agencé pour être monté de manière amovible sur le réceptacle 4 et destiné à fermer au moins en partie une première extrémité 41 du réceptacle 4. De préférence, le couvercle 7 est souple et élastique, à la manière d'une membrane. Le réceptacle 4 comprend à sa première extrémité 41 des saillies 42 destinées à coopérer avec des sillons 71 correspondants que présente le couvercle 7. De préférence, le couvercle 7 comprend en outre une languette 73 agencée pour faciliter le montage et le démontage du couvercle 7 sur le réceptacle 4 et en particulier l'engagement des saillies 42 dans les sillons 71 en déformant/étirant le couvercle 7.

Le couvercle 7 présente une ouverture centrale 8 dont la taille est suffisamment grande pour permettre le passage de l'instrument dentaire sans que l'instrument ne touche les parois de l'ouverture 8. A titre d'exemple, les instruments dentaires classiques ont une partie active dont le diamètre varie entre quelques centièmes de millimètre et moins de 3 millimètres. Le couvercle 7 présente en outre au moins une fente 9 communicant avec l'ouverture 8. Tel qu'illustré, le couvercle 7 comprend dix fentes 9 toutes ouvertes à une de leur extrémité sur l'ouverture centrale 8. Chaque fente 9 est dimensionnée de sorte qu'il est nécessaire d'exercer une force sur l'instrument dentaire pour le faire passer au travers de la fente 9 et de sorte que sa partie active frotte contre les parois de la fente lorsqu'elle la traverse. Ainsi, en faisant passer l'instrument dentaire au travers de la fente essentiellement perpendiculairement à celle-ci, il est possible de nettoyer mécaniquement la partie active, les débris ou les liquide plus ou moins visqueux présents sur ladite partie active étant retenus par la fente 9.

De préférence, les fentes 9 sont en outre dimensionnées pour permettre de retenir l'instrument dentaire et notamment sa première portion et servir ainsi de logement aux instruments dentaires pour leur mise à disposition durant un traitement dentaire.

De préférence, le couvercle 7 est obtenu par moulage par injection dans un matériau qui lui permet d'être relativement souple comme le TPE ou le silicone. Dans la forme d'exécution illustrée aux figures, le couvercle 7 présente une épaisseur plus importante sur sa périphérie, là où se trouve les sillons 71, tandis que l'épaisseur de la portion centrale dans laquelle sont prévues l'ouverture centrale 8 et les fentes 9 est plus fine. De plus, les fentes 9 présentent des rebords 91 dont l'épaisseur est la plus fine du couvercle 7 de sorte que ces rebords 91 forment des lames souples permettant d'améliorer encore le nettoyage de la partie active d'un instrument dentaire à la manière d'une raclette à vitre.

De préférence, le dispositif 1 comprend en outre une règle 6 agencée sur le l'anneau 3 pour permettre de mesurer la longueur active d'un instrument dentaire ou encore la longueur d'un cône de gutta percha. De préférence, la règle 6 comprend une graduation 61 en millimètres agencée pour pouvoir être utilisée de droite à gauche ou de gauche à droite selon la main ou le côté de l'utilisateur où se trouve le dispositif 1. De préférence, la règle 6 comprend à chaque extrémité de la graduation 61, un trou 62 dans lequel peut venir se loger une portion d'un élément stoppeur destiné à être glissé le long de l'instrument dentaire pour délimiter la longueur de la portion de travail de la partie active de l'instrument. Grâce au trou 62 et à la graduation 61, il est donc possible de positionner un élément stoppeur précisément le long de l'instrument et notamment de sa partie active :
- l'élément stoppeur est d'abord enfilé sur l'instrument (soit par l'utilisateur au moment du traitement soit par le fabricant de l'instrument), notamment sur sa partie active ;
- il est ensuite positionné dans un des trous 62 (selon la main ou le côté où se trouve le dispositif 1) ;
- avec l'élément stoppeur bloqué dans le trou 62, il est possible de faire glisser l'instrument à travers ledit élément stoppeur en regard de la graduation 61 jusqu'à ce que ledit élément stoppeur soit positionné à la bonne longueur sur la partie active de l'instrument.

De préférence et tel qu'illustré aux figures, l'anneau 3, le réceptacle 4 et la règle 6 sont venus d'une seule pièce qui peut être réalisée dans tout matériau approprié mais de préférence en plastique. En variante, ces pièces pourraient être rapportées les unes aux autres pour former le dispositif 1 et être réalisées dans des matériaux distincts.

Pour utiliser le dispositif 1, l'utilisateur vient placer le couvercle 7 sur l'extrémité 41 du réceptacle 4. Dans la forme d'exécution illustrée, cette opération est facilitée par la languette 73, l'agencement des saillies 42 et des sillons 71 et par le fait que le couvercle 7 est souple. Il est en effet possible de positionner une partie des sillons 71 dans les saillies 42, puis de terminer de mettre en place le couvercle 7 en le déformant en tirant sur la languette 73 pour que toutes les saillies 42 prennent place dans les sillons correspondants 71. Le couvercle 7 est alors monté sur le réceptacle 4.

A ce stade, il est déjà possible de venir positionner les instruments dentaires prévus pour un traitement particulier sur le dispositif 1. Pour ce faire, l'utilisateur manipule l'instrument par sa première portion et l'insère dans le réceptacle 4 par l'ouverture centrale 8 du couvercle 7 dans une direction parallèle à l'axe longitudinal du réceptacle 4. Une fois que l'entier de la partie active de l'instrument se trouve dans le réceptacle 4, l'instrument est ensuite déplacé/glissé dans une fente 9 du couvercle 7 dans une direction parallèle à l'axe longitudinal de ladite fente 9 pour être retenu dans cette fente 9. De préférence, c'est la première portion de l'instrument, celle qui n'est pas active, qui est retenu dans la fente 9. Comme on le verra plus loin, cela permet d'assurer un nettoyage complet de toute la partie active. Ainsi, grâce à l'ouverture centrale 8 du couvercle 7, il n'y a aucun effort exercé sur la pointe très fragile de l'instrument lorsque celui-ci est inséré dans le réceptacle 4 du dispositif 1. Les fentes 9 permettent quant à elles de servir de support aux instruments pour leur mise à disposition confortable et efficiente durant le traitement. De plus, les fentes 9 offrent des positions bien définies qui permettent d'identifier clairement la place d'un instrument dans une séquence ou l'état d'un instrument (utilisé ou non, propre ou sale par exemple).

Ensuite, l'utilisateur peut positionner le dispositif 1 sur un de ses doigts, en général l'index de la main gauche pour les droitiers et de la main droite pour les gauchers, en glissant le doigt choisi dans la bague 3.

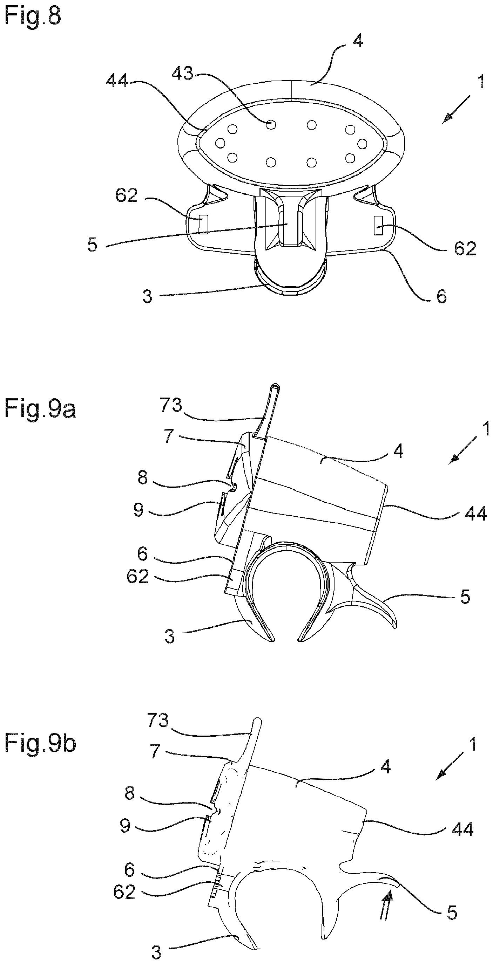

En position de service du dispositif 1 sur un doigt de l'utilisateur, l'extension 5 vient s'appuyer sur le doigt adjacent à celui sur lequel est enfilée la bague 3 et évite ainsi au dispositif 1 de tourner. De préférence et tel qu'illustré aux figures 9a et 9b, l'extension 5 et l'anneau 3 permettent également une mise en place facilité du dispositif 1 sur le doigt de l'utilisateur. En effet, l'anneau 3 et l'extension 5 sont de préférence conformés pour que l'utilisateur puisse exercer une force sur l'extension 5 de sorte à déformer l'anneau 3 pour l'agrandir comme illustré sur la figure 9b. Il est ainsi plus facile pour l'utilisateur de positionner le dispositif 1 et d'enfiler l'anneau 3 sur le doigt de son choix. Cette déformation de l'anneau 3 est illustrée aux figures 9a (position de repos) et 9b (position déformée).

Une fois le dispositif positionné sur le doigt de l'utilisateur, ledit dispositif 1 est prêt à être utilisé. Soit le dispositif 1 est déjà équipé du ou des instruments nécessaires au traitement dentaire envisagé, soit l'utilisateur peut aussi positionner les instruments dans le dispositif 1 à ce moment-là, une fois le dispositif 1 en place sur sa main, selon la procédure décrite ci-dessus.

Durant le traitement, l'utilisateur a accès facilement aux instruments dont il a besoin : il peut retirer l'instrument adéquat simplement en le tirant hors du réceptacle 4 à travers la fente 9. De plus, l'utilisateur peut également se servir de la règle 6 pour mesurer la longueur adéquate de la partie active de l'instrument et/ou placer ou régler la position d'un élément stoppeur sur ladite partie active au moyen des trous 62 pour ainsi définir la longueur de travail idoine de la partie active.

Pour nettoyer un instrument sale, l'utilisateur va insérer l'instrument dans le réceptacle 4 via l'ouverture centrale 8 du couvercle 7. Ainsi, vue la forme et la taille de cette ouverture centrale 8, aucun effort n'est exercé sur la pointe fragile de l'instrument, et aucun débris ne remonte le long de l'instrument puisque ledit instrument, et notamment sa partie active, ne vont pas toucher les bords de l'ouverture centrale 8. Ensuite, l'utilisateur déplace l'instrument en translation dans le réceptacle 4 dans une des fentes 9. De préférence, l'entier de la partie active de l'instrument est inséré dans le réceptacle 4 de sorte que c'est la première portion de l'instrument, celle qui n'est pas active, qui vient au contact de la fente 9 durant la translation de l'instrument. Ainsi, on évite d'abîmer la partie active et/ou la fente durant cette translation et la partie active de l'instrument, celle comprenant des débris, se trouve entièrement dans le réceptacle 4 sous une fente 9 du couvercle 7. En tirant l'instrument au travers de la fente 9 pour faire sortir la partie active du réceptacle 4, les parois de la fente 9 frottent contre ladite partie active et exercent leur action de nettoyage mécanique. Plusieurs passages à travers une fente 9 sont bien entendu possibles pour garantir un bon nettoyage. Les débris solides ou liquides enlevés restent prisonniers sous le couvercle 7 et dans le réceptacle 4 et ne risquent pas de contaminer à nouveau la partie active d'un instrument lors de nettoyages subséquents.

De préférence, le dispositif 1 est conçu pour que, à la fin du traitement, le corps principal venu d'une pièce et formé de l'anneau 3, de l'extension 5, du réceptacle 4 et de la règle 6 puisse être nettoyé et stérilisé pour une prochaine utilisation. De manière privilégiée, le corps principal est conçu pour résister à plusieurs dizaines de stérilisation. Le couvercle 7 quant à lui est de préférence jetable. En variante, il pourrait également être conçu pour une réutilisation après stérilisation.

Le dispositif 1 décrit ci-dessus est particulièrement bien adapté pour les instruments endodontiques manuels ou rotatifs (dont la première portion est destinée à être fixée dans un contre-angle pour leur mise en rotation). Ces instruments destinés à être fixés dans un manche auxiliaire ont donc une dimension particulièrement adaptée pour être stockés dans le dispositif selon l'invention. En ce qui concerne les curettes ou autre détartreur dont la première portion prend souvent la forme d'un manche que l'utilisateur peut prendre en main lors du traitement, bien qu'il ne soit pas possible de stocker ces instruments dans le dispositif selon l'invention de par la taille de l'instrument et notamment sa longueur, il est toujours possible de nettoyer la partie active de ces instruments en l'insérant dans le réceptacle 4 et en la passant à travers les fentes 9 du couvercle pour ainsi enlever mécaniquement les débris liquides ou solides. Dans ce sens, le dispositif selon l'invention permet d'abord le nettoyage des instruments dentaires, médicaux ou chirurgicaux et selon la taille des instruments, permet aussi leur stockage.

Le dispositif 1 pourrait en outre comprendre tout support ou accessoires utiles lors d'un traitement dentaire, médical ou chirurgical comme un contenant pour cône de gutta percha ou pour ciment d'obturation ou pour une solution désinfectante (par exemple imbibée sur une mousse). Une telle mousse imbibée de désinfectant pourrait également se trouver à l'intérieur du réceptacle 4. Ledit réceptacle 4 pourrait être entièrement fermé à sa seconde extrémité 44 opposée à la première 41 de sorte à retenir un liquide désinfectant.

La figure 8 illustre une variante de la forme d'exécution ci-dessus dans laquelle le réceptacle 4 est partiellement fermé à sa seconde extrémité 44 opposée à la première extrémité 41 et présente des ouvertures 43 facilitant le nettoyage et la stérilisation.

En variante également, l'anneau 3 pourrait être fermé ou agencé pour avoir une taille réglable par déformation ou par tout autre moyen d'ajustement approprié.

De préférence, le dispositif 1 est agencé pour pouvoir être posé sur une surface plate de sorte que les instruments logés dans les fentes 9 du couvercle 7 ne touchent pas ladite surface.

Dans la forme d'exécution illustrée aux figures, le couvercle 7 présente une ouverture centrale 8 communicant avec chacune des fentes 9. En variante, le couvercle 7 pourrait présenter plusieurs ouvertures de plus grand diamètre que les fentes 9 communicant chacune avec une ou plusieurs desdites fentes 9. Par exemple, chaque fente 9 pourrait se terminer par une ouverture plus large à travers laquelle la partie active d'un instrument peut passer sans accrocher les parois de ladite ouverture.

Si la ou les ouvertures du couvercle ont pour avantage de réduire les risques de casse de la partie active de l'instrument et de limiter la remontée de débris le long de l'instrument lors de l'insertion de celui-ci dans le réceptacle à travers le couvercle, ces ouvertures ne sont pas essentielles pour les fonctions de stockage et de nettoyage des instruments. En effet, en variante, le couvercle 7 pourrait ne comprendre qu'une ou des fentes 9. Dans ce cas, les instruments sont insérés dans le réceptacle 4 par les fentes 9 directement, le nettoyage et le stockage des instruments dans la fente 9 fonctionnant toujours sur le principe décrit ci-dessus.

Le dispositif tel qu'illustré aux figures pourrait également s'utiliser sans le couvercle 7 pour stocker des cônes de gutta percha dans le réceptacle 4 et les mesurer à l'aide de la règle 6.

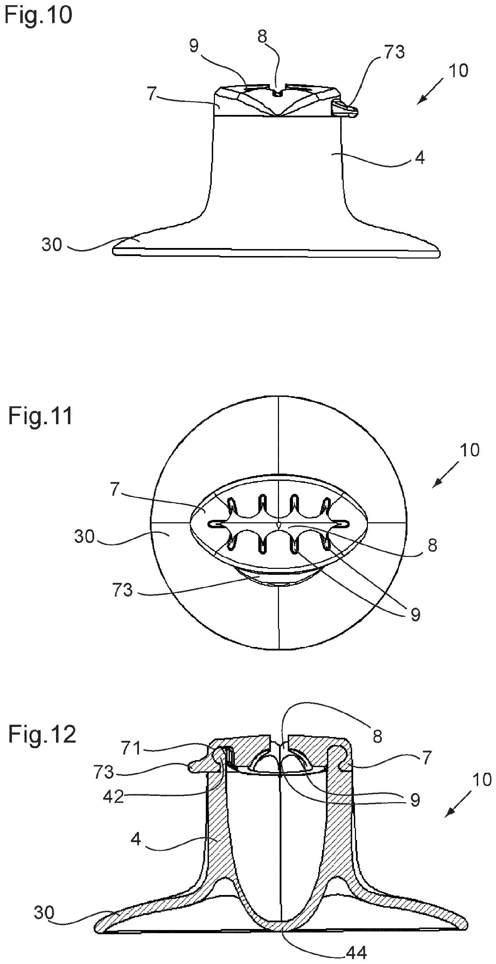

Les figures 10 à 12 illustrent une seconde forme d'exécution du dispositif de nettoyage pour instruments dentaires, médicaux ou chirurgicaux selon l'invention. Les éléments de cette forme d'exécution identiques ou quasi-identiques à ceux de la première forme d'exécution portent les mêmes références.

Dans cette forme d'exécution, le dispositif de nettoyage 10 est destiné à être posé sur une surface essentiellement plate comme un plateau ou un champ opératoire. Pour ce faire, le support du dispositif 10 - qui avait la forme d'une bague dans la première forme d'exécution - a la forme d'un socle 30 dans cette seconde forme d'exécution.

Comme dans la première forme d'exécution, le réceptacle 4 est monté sur le socle 30 et le tout est de préférence venu d'une seule pièce. En variante, le dispositif 10 pourrait aussi comprendre une règle 6 semblable à celle décrite dans la première forme d'exécution et qui pourrait soit être montée sur le réceptacle 4 ou le socle 30 soit être venue d'une seule pièce avec ces deux derniers éléments. Dans la forme d'exécution illustrée, le réceptacle 4 est fermé à son extrémité 44 opposée à celle recevant le couvercle 7.

Les caractéristiques du couvercle 7 et le fonctionnement du dispositif 10 sont en tout point similaires à ceux décrits en référence à la première forme d'exécution.

De préférence, le socle 30 comprend une portion anti-dérapante pour garantir une bonne adhérence avec la surface sur laquelle il est posé. En variante, le socle pourrait comprendre tout moyen approprié permettant d'assurer cette adhérence, comme une portion en silicone ou TPE, une ou plusieurs ventouses, un ou plusieurs pieds ou une forme particulière.

Toutes les variantes et avantages décrits ci-dessus en référence à la première forme d'exécution restent valables pour cette seconde forme d'exécution. De plus, le fonctionnement et l'utilisation du dispositif 10 selon la seconde forme d'exécution est en outre similaire à ce qui a été décrit ci-dessus.

Les figures 13 à 17 illustrent une troisième forme d'exécution de l'invention. Dans cette forme d'exécution, le dispositif de nettoyage 100 est destiné à être posé sur une surface essentiellement plate comme un plateau ou un champ opératoire. Pour ce faire, le support du dispositif 100 - qui avait la forme d'une bague dans la première forme d'exécution - a la forme d'un socle 300 similaire à celui de la seconde forme d'exécution.

De préférence, le socle 300 comprend une portion anti-dérapante pour garantir une bonne adhérence avec la surface sur laquelle il est posé. Dans la forme d'exécution illustrée, cette portion anti-dérapante est constituée par un joint O-ring 301 logé dans une rainure 302 prévue à cet effet sur le fond du socle 300. En variante, le socle pourrait comprendre tout moyen approprié permettant d'assurer cette adhérence, comme une portion en silicone ou TPE, une ou plusieurs ventouses, un ou plusieurs pieds ou une forme particulière.

Comme dans la première forme d'exécution, le réceptacle 400 est monté sur le socle 300 et le tout est de préférence venu d'une seule pièce. Comme dans la seconde forme d'exécution, le réceptacle 400 est fermé à son extrémité 440 opposée à celle recevant le couvercle 700 (extrémité côté support 300).

Une des particularités de cette troisième forme d'exécution réside dans le couvercle 700 qui est réalisé en deux parties. En effet, le couvercle 700 selon cette troisième forme d'exécution comprend une première partie 701 ayant la forme d'un opercule, de préférence souple et élastique à la manière d'une membrane. Cette première partie 701 est destinée à venir se poser sur la première extrémité 410 du réceptacle 400 pour fermer celui-ci. De préférence, cette première partie 701 comprend des moyens de centrage permettant d'assurer son positionnement sur ladite première extrémité 410 du réceptacle 400. Dans l'exemple illustré en particulier à la figure 14, la première partie 701 comprend une rainure 7011 sur sa partie inférieure ainsi qu'un rebord 7012 à sa périphérie destinés à coopérer avec les bords de la première extrémité 410 du réceptacle 400 pour le positionnement et en particulier le centrage de la première partie 701 du couvercle 700 lorsqu'elle est posée sur ledit réceptacle 400. Tout autre moyen de centrage ou de fixation de la première partie 701 du couvercle 700 sur le réceptacle 400 pourrait être prévu, étant entendu que ces moyens sont totalement optionnels.

Le couvercle 700 comprend en outre une seconde partie 702 qui vient se positionner sur le réceptacle 400 pour maintenir la première partie 701 du couvercle 700 sur la première extrémité 410 du réceptacle 400. Comme illustré au figures 13 à 17, la seconde partie 702 a la forme d'une bague avec une ouverture centrale 7021 à travers laquelle est accessible la première partie 701 du couvercle 700 en position assemblée du dispositif 100, un rebord 7022 qui peut par exemple porter une règle 60 similaire à la règle 6 décrite en référence à la première forme d'exécution, et une portion annulaire 7023 destinée à vernir entourer le réceptacle 400 pour la fixation de cette seconde partie 702 et donc du couvercle 700 sur ledit réceptacle 400.

Comme dans les formes d'exécution précédentes, le couvercle 700 comprend une ouverture centrale 800 et des fentes 900. En particulier, dans cette troisième forme d'exécution, l'ouverture centrale 800 et les fentes 900 sont réalisées dans la première partie 701 du couvercle 700 et sont accessibles à travers l'ouverture 7021 de la deuxième partie 702 du couvercle 700 lorsque le dispositif 100 est assemblé. Les caractéristiques et fonctionnement de ces fentes et ouverture centrale sont en tout point similaires à ce qui a été décrit ci-dessus en référence avec la première forme d'exécution.

De préférence, la première partie 701 du couvercle 700 est réalisée dans un matériau souple et élastique. Cette première partie 701 peut notamment être réalisée en élastomère thermoplastique (TPE), en silicone ou dans une mousse, par exemple une mousse à structure sandwich. Quant à la deuxième partie 702 du couvercle 700, celle-ci peut être réalisée en acier inoxydable, en matériau thermoplastique ou thermodurcissable.

Dans cette forme d'exécution, la première partie 701 du couvercle peut être jetable tandis que la deuxième partie 702 est de préférence stérilisable et réutilisable.

Le couvercle 700 est assemblé sur le réceptacle 400 de la façon suivante : avec le réceptacle 400 et le support 300 posé sur une surface plate (table, plateau), la première partie 701 du couvercle 700 est posée sur la première extrémité 410 du réceptacle 400, éventuellement positionnée à l'aide de la rainure 7011 et/ou des rebords 7012. La deuxième partie 702 est ensuite insérée sur le réceptacle 400 pour maintenir la première partie 701 en position. En général, la deuxième partie 702 est enfoncée sur le réceptacle 400 jusqu'à venir en butée sur la première extrémité 410 de ce réceptacle. Pour assurer l'assemblage du couvercle 700 sur le réceptacle 400, ledit réceptacle 400 comprend un épaulement 401 autour duquel la deuxième partie 702 du couvercle 700 vient se chasser à force. Cela permet de pouvoir manipuler l'entier du dispositif 100 en le prenant par ledit couvercle 700 (en particulier par la deuxième partie 702). En variante, le réceptacle et le couvercle pourraient comprendre des moyens d'encliquetage ou tout autre moyen approprié permettant l'assemblage de la deuxième partie du couvercle sur le réceptacle.

Une fois le couvercle 700 assemblé sur le réceptacle 400, les fentes 900 et/ou l'ouverture 800 de la première partie 701 du couvercle 700 sont accessibles à travers l'ouverture 7021 de la deuxième partie 702 du couvercle 700. Le nettoyage d'un instrument sale ou un le stockage d'un instrument à l'aide du dispositif 100 selon la troisième forme d'exécution peut alors se faire de la même façon qu'avec un dispositif 1 ou 10 selon l'une des première ou seconde forme d'exécution décrites ci-dessus.

Toutes les variantes et avantages décrits ci-dessus en référence aux première et seconde formes d'exécution restent valables pour cette troisième forme d'exécution.

Outre la règle 60 qui se trouve sur la deuxième partie 702 du couvercle 700, les figures 13 à 17 illustrent encore des moyens complémentaires de mesure de la longueur utile d'un instrument dentaire particulièrement pratiques et faciles d'utilisation. En effet, le dispositif 100 selon la troisième forme d'exécution comprend en outre une échelle de mesure 600 agencée pour être mobile en rotation autour du réceptacle 400 et/ou du couvercle 700 tout en étant bloquée en translation par rapport audit réceptacle 400 lorsque le couvercle 700 est assemblé sur le réceptacle 400. Comme illustré aux figures, cette échelle de mesure 600 a la forme d'un escalier et comprend plusieurs marches 601 correspondant chacune à une longueur pour former ainsi une graduation. Des indications de longueur 602 peuvent être prévues sur chacune des marches ou sur le côté de l'échelle de mesure 600.

Dans la forme d'exécution illustrée, la deuxième partie 702 du couvercle 700 est agencée pour venir en butée par sa portion annulaire 7023 contre le support 300 lorsque le couvercle 700 est monté sur le dispositif 100. Cette portion annulaire 7023 comprend à son extrémité opposée au rebord 7022 un premier épaulement 7024 agencée pour coopérer avec un second épaulement 603 que comprend l'échelle de mesure 600 sur sa surface intérieure pour bloquer celle-ci en translation sur le réceptacle 400 (voir la figure 15).

Pour utiliser cette échelle de mesure 600, le couvercle 700 comprend un trou 7025 sur le rebord 7022 de sa deuxième partie 702. L'utilisateur qui veut déterminer la longueur adéquate de la partie active d'un instrument et en particulier positionner un élément stoppeur le long de cette partie active peut alors :
- pivoter l'échelle de mesure 600 jusqu'à placer la marche 601 de la longueur souhaitée sous le trou 7025 (un indicateur peut être prévu sur la partie annulaire 7023 de la seconde partie 702 du couvercle 700 ;
- insérer ledit instrument dans le trou 7025 et éventuellement, aligner l'axe de l'instrument avec un axe dessiné sur l'extérieur de la portion annulaire 7023 du couvercle 700 pour garantir la verticalité (cet axe peut correspondre à l'indicateur mentionné ci-dessus ;
- déplacer l'élément stoppeur le long de la partie active de l'instrument en poussant sur celui-ci jusqu'à ce que la pointe touche la marche 601 choisie, ledit élément stoppeur est alors positionné à la longueur souhaitée sur la partie active de l'instrument.

Les moyens de mesure formés par l'échelle de mesure 600 et le trou 7025 peuvent également servir à mesurer la longueur d'un cône de gutta percha et couper ledit cône à la longueur désirée.

Ainsi, ces moyens de mesures permettent d'ajuster facilement la longueur de la partie active d'un instrument en limitant le nombre d'opérations : un seul trou dans lequel insérer l'instrument, puis l'échelle de mesure qui pivote. Ceci facilite également l'étape de désinfection du dispositif de nettoyage 100.

En variante, l'échelle de mesure 600 et/ou le réceptacle 400 et/ou le couvercle 700 peuvent comprendre des moyens d'indexation agencés pour déterminer des positions d'indexage de l'échelle de mesure dans lesquelles une marche 601 de l'échelle est alignée avec le trous 7025 du couvercle 700.

De préférence, l'échelle de mesure 600 est réalisée en acier inoxydable ou dans un matériau thermoplastique (TPE/POM).

De manière générale, le dispositif selon l'invention de nettoyage pour instruments dentaires, médicaux ou chirurgicaux lors d'un traitement dentaire, médical ou chirurgical comprend :
- Un support ;
- Un réceptacle monté sur le support et destiné à recevoir au moins une portion de la partie active d'un instrument dentaire, médical ou chirurgical ; et
- Un couvercle agencé pour être monté de manière amovible sur une extrémité du réceptacle.

Le couvercle comprend au moins une fente. Ladite fente est agencée de sorte que la partie active de l'instrument peut être insérée dans le réceptacle à travers la fente et le frottement entre la partie active de l'instrument et au moins une paroi de la fente lorsque ledit instrument traverse ladite fente permet de nettoyer mécaniquement ladite partie active.

De préférence, la fente est également conformée pour retenir l'instrument dans le réceptacle et servir de support à celui-ci lorsque sa partie active est insérée dans le réceptacle.

De préférence, le couvercle comprend en outre au moins une ouverture communicant avec la ou les fentes et plus large que celle-ci et à travers laquelle l'instrument et notamment sa partie active peuvent passer sans frottement et sans contrainte.

Selon une première variante, le support est un anneau ouvert ou fermé ou de taille réglable destiné à être enfilé sur le doigt d'un utilisateur.

De préférence, le dispositif comprend une extension solidaire de l'anneau et agencée pour venir s'appuyer sur le doigt adjacent au doigt passant à travers l'anneau lorsque le dispositif est en place de manière à empêcher l'anneau et le dispositif de tourner.

Selon une seconde variante, le support est un socle destiné à être posé sur une surface essentiellement plane, telle un plateau ou un champ opératoire.

Le réceptacle peut être totalement ou partiellement fermé à son extrémité opposée à celle recevant le couvercle. Le réceptacle peut également contenir des moyens désinfectants comme une mousse imbibée d'une solution désinfectante.

Le dispositif peut comprendre en outre une règle montée sur le réceptacle ou le support et comportant une graduation permettant de mesurer la longueur de la portion de travail de la partie active d'un instrument. De préférence, un logement borgne ou traversant (trou) est agencé au début de ladite graduation

De préférence, le couvercle comprend plusieurs fentes qui peuvent être disposées pour former des secteurs ou groupes permettant de classer ou trier plusieurs instruments selon des critères déterminés (sale et propre, ou encore selon la séquence utilisée pour le traitement).

De préférence, le couvercle est souple et élastique, réalisé par exemple en plastique, permettant ainsi de se déformer pour sa mise en place sur le réceptacle. De plus, de manière privilégiée, il présente une languette permettant de monter et démonter ledit couvercle facilement sur le réceptacle.

De préférence, le support, le réceptacle, la règle et/ou l'extension sont venus d'une seule pièce. La pièce est par exemple réalisée en plastique ou en élastomère et est stérilisable.

Selon les variantes, le couvercle est venu d'une ou plusieurs pièces.

Selon certaines variantes, le dispositif comprend en outre des moyens de mesure comprenant un trou dans une portion du dispositif dans lequel une tige effilée (instrument ou cône) peut être insérée sans pénétrer dans le réceptacle et une échelle de mesure agencée pour être mobile en rotation mais fixe en translation par rapport au réceptacle, l'échelle de mesure comprenant plusieurs marches déterminant chacune une longueur particulière.

On réalise ainsi un dispositif de nettoyage pour instruments dentaires, médicaux ou chirurgicaux offrant une aide cruciale et efficace pour le nettoyage et/ou la mise à disposition d'instruments durant un traitement dentaire, médical ou chirurgical. La ou les fentes du couvercle assurent un nettoyage mécanique efficace tout en limitant les risques de casse ou de déformation des instruments. Ce risque est encore plus faible avec une ouverture large au travers de laquelle la pointe fragile des instruments peut passer sans frottement ni contrainte. Dans cette variante également, le nettoyage est amélioré puisqu'il est possible d'empêcher la remontée des débris le long de l'instrument lorsque l'instrument est inséré dans le réceptacle.

La présence de fentes sur le couvercle permet un agencement clair des instruments et permet de distinguer les instruments selon des critères définis comme la saleté, l'ordre d'utilisation durant le traitement... Cet agencement ordonné est difficilement réalisable avec un dispositif comprenant un coussinet en mousse tel que l'on connait.

Le dispositif est en outre ergonomique, facile à prendre en main, polyvalent dans le sens où il peut être utilisé pour un grand nombre de traitements et d'instruments : endodontique, parodontiques ou autre traitement médicaux, limes, fraise, curette, détartreur, cône de gutta percha...

Le dispositif fournit une aide efficace à l'utilisateur pour le nettoyage et/ou la mise à disposition des instruments durant un traitement dentaire, médical ou chirurgical et permet d'augmenter sensiblement l'efficience dudit traitement.

Enfin, chaque élément peut être réalisé dans des matériaux facilement usinables et par des procédés simples et connus ce qui rend le dispositif avantageux commercialement.

## Revendications

1. Dispositif (1 ;10) de nettoyage pour instruments dentaires, médicaux ou chirurgicaux comprenant une partie active, ledit dispositif comprenant :
• Un support (3 ; 30) ;
• Un réceptacle (4) monté sur le support (3 ;30) et destiné à recevoir au moins la partie active d'un instrument dentaire, médical ou chirurgical ; et
• Un couvercle (7) agencé pour être monté de manière amovible sur une extrémité (41) du réceptacle (4) ;
et dans lequel le couvercle (7) comprend au moins une fente (9), ladite fente (9) étant agencée de sorte que au moins la partie active de l'instrument peut être insérée dans le réceptacle (4) et retirée du réceptacle (4) à travers la fente (9), le frottement entre la partie active de l'instrument et au moins une paroi de la fente (9) lorsque ledit instrument traverse ladite fente (9) permettant de nettoyer mécaniquement ladite partie active en retenant débris et saletés liquides ou solides.

2. Dispositif (1) selon la revendication 1, dans lequel le support est un anneau (3) destiné à recevoir un doigt d'un utilisateur, l'anneau (3) étant ouvert ou fermé ou de taille réglable.

3. Dispositif (1) selon l'une des revendications 1 ou 2, dans lequel une extension (5) solidaire de l'anneau (3) est agencée pour venir s'appuyer sur le doigt adjacent au doigt passant à travers l'anneau (3) lorsque le dispositif est en place de manière à empêcher l'anneau (3) et le dispositif (1) de tourner en position de service du dispositif (1).

4. Dispositif (10) selon la revendication 1 dans lequel le support est un socle (30) destiné à être posé sur une surface essentiellement plane.

5. Dispositif selon l'une des revendications précédentes, dans lequel le couvercle (700) est formé de plusieurs pièces (701, 702).

6. Dispositif selon la revendication précédente, dans lequel au moins une pièce du couvercle à la forme d'un opercule réalisé dans une mousse fine, ledit opercule présentant ladite au moins une fente (900).

7. Dispositif (1 ; 10) selon l'une des revendications précédentes, dans lequel la fente (9) est également conformée pour retenir l'instrument et servir de support à celui-ci lorsque la partie active est insérée dans le réceptacle (4) à travers la fente (9), une force exercée sur l'instrument étant nécessaire pour faire passer ledit instrument à travers la fente (9).

8. Dispositif (1 ; 10) selon l'une des revendications précédentes, dans lequel le couvercle (7) comprend en outre au moins une ouverture (8) communicant avec la ou les fentes (9) et plus large que celle-ci et à travers laquelle l'instrument peut passer sans frottement et sans contrainte.

9. Dispositif (1 ; 10) selon l'une des revendications précédentes, dans lequel le réceptacle (4) est au moins partiellement fermé à son extrémité opposée à celle recevant le couvercle (7)

10. Dispositif (1 ; 10) selon la revendication précédente, dans lequel le réceptacle (4) contient des moyens désinfectants comme une solution désinfectante ou une mousse imbibée d'une telle solution.

11. Dispositif (1 ; 10) selon l'une des revendications précédentes, dans lequel une règle (6) est montée sur le réceptacle (4) ou le support (3 ; 30) et comprend une graduation (61).

12. Dispositif (1 ; 10) selon l'une des revendications précédentes, dans lequel le couvercle (7) comprend plusieurs fentes (9) qui peuvent être disposées pour former des secteurs ou groupes permettant de classer ou trier plusieurs instruments selon des critères déterminés .

13. Dispositif (1 ; 10) selon l'une des revendications précédentes, dans lequel le couvercle (7) est souple et élastique.

14. Dispositif (1 ; 10) selon la revendication précédente, dans lequel le couvercle (7) présente une languette (73) permettant de monter et démonter ledit couvercle (7) facilement sur le réceptacle (4) en déformant ledit couvercle (7).

15. Dispositif (1 ; 10) selon l'une des revendications précédentes, dans lequel le support (3 ; 30), le réceptacle (4), la règle (6) et/ou l'extension (5) sont venus d'une seule pièce pour former un corps principal.
